# EUROPÄISCHE PATENTSCHRIFT

(11) **EP 1 529 027 B1**
(45) Veröffentlichungstag und Bekanntmachung des Hinweises auf die Patenterteilung: **29.08.2007**
(21) Anmeldenummer: 03790870.4
(22) Anmeldetag: 06.08.2003
(51) Int. Cl.: C07C 209/84, C07C 211/36

(54) **VERFAHREN ZUR HERSTELLUNG VON ISOPHORONDIAMIN (IPDA, 3-AMINOMETHYL-3,5,5-TRIMETHYLCYCLOHEXYLAMIN) MIT EINEM HOHEN CIS/TRANS-ISOMERENVERH LTNIS**
METHOD FOR THE PRODUCTION OF ISOPHORONDIAMINE (IPDA, 3-AMINOMETHYL-3,5,5-TRIMETHYLCYCLOHEXYLAMINE) HAVING A HIGH CIS/TRANS-ISOMER RATIO
PROCEDE POUR PRODUIRE DE L'ISOPHORONDIAMINE (IPDA, 3-AMINOMETHYL-3,5,5-TRIMETHYLCYCLOHEXYLAMINE) A RAPPORT D'ISOMERES CIS/TRANS ELEVE

(30) Priorität: 09.08.2002 DE 10236675
(43) Veröffentlichungstag der Anmeldung: 11.05.2005
(73) Patentinhaber: BASF Aktiengesellschaft, 67056 Ludwigshafen (DE)
(72) Erfinder: FUNKE, Frank, 67067 Ludwigshafen (DE); HILL, Thomas, 67071 Ludwigshafen (DE); VON WATZDORF, Jobst, Rüdiger, 68163 Mannheim (DE); MATTMANN, Wolfgang, 67117 Limburgerhof (DE); HARDER, Wolfgang, 69469 Weinheim (DE); HENKES, Erhard, 64683 Einhausen (DE); LITTMANN, Gerd, 69469 Weinheim (DE); JULIUS, Manfred, 67117 Limburgerhof (DE)
(74) Vertreter: Isenbruck, Günter
(86) Internationale Anmeldenummer: PCT/EP2003/008722
(87) Internationale Veröffentlichungsnummer: WO 2004/020386

(56) Entgegenhaltungen:
- US-A- 5 756 845
- US-A- 6 022 999

## Beschreibung

Die Erfindung betrifft Verfahren zur Herstellung von 3-Aminomethyl-3,5,5-trimethylcyclohexylamin (Isophorondiamin, IPDA) mit einem hohen cis/trans-Isomerenverhältnis.

IPDA wird als Ausgangsprodukt zur Herstellung von Isophorondiisocyanat (IPDI), einer Isocyanatkomponente für Polyurethansysteme, als Aminkomponente für Polyamide und als Härter für Epoxidharze verwendet. IPDA wird üblicherweise aus 3-Cyano-3,5,5-trimethylcyclohexanon (Isophoronnitril, IPN) hergestellt, wobei in Gegenwart von Ammoniak, Wasserstoff und üblichen Hydrierkatalysatoren die Carbonylgruppe in eine Aminogruppe und die Nitrilgruppe in eine Aminomethylgruppe überführt werden. Man erhält Gemische aus cis-IPDA und trans-IPDA. Beide Isomere weisen unterschiedliche Reaktivitäten auf, was für die vorgesehene technische Anwendung von Bedeutung ist. Gemäß DE-A 42 11 454 werden durch Verwendung eines IPDA-Isomerengemischs, bestehend aus über 40% des trans-Isomeren und unter 60% des cis-Isomeren, als Reaktionskomponente in Polyadditionsharzen, wie insbesondere Epoxidharzen, sowohl die Topfzeit verlängert als auch die maximale Härtungstemperatur erniedrigt. Zur Erzielung einer möglichst hohen Reaktionsgeschwindigkeit werden umgekehrt IPDA-Isomerengemische bevorzugt, welche einen möglichst hohen Anteil an dem cis-Isomeren (≥ 70%) aufweisen. Kommerziell erhältliches IPDA besitzt deshalb ein cis/trans-Isomerenverhältnis von 75/25.

Unterschiedliche Verfahren zur Erzielung eines hohen cis/trans- bzw. eines hohen trans/cis-Verhältnisses sind aus dem Stand der Technik bereits bekannt.

Gemäß der DE-A 43 43 890 erfolgt die aminierende Hydrierung des IPN zum IPDA, indem man ein Gemisch aus IPN, Ammoniak und einem C₁-C₃-Alkohol in Gegenwart von Wasserstoff über einen mit einem Cobalt- und/oder Ruthenium-Festbettkatalysator ausgestatteten Rieselbettreaktor bei 3 bis 8 MPa und einer Temperatur von 40 bis 150°C, vorzugsweise 90 bis 130°C, rieseln lässt und das Reaktionsgemisch zur Abtrennung von NH₃, H₂O und Nebenprodukten destillativ aufarbeitet. Bei Verwendung eines Ru-Trägerkatalysators werden hohe cis/trans-Isomerenverhältnisse von 84/16 (Gesamtausbeute an IPDA: 81%) erzielt.

Die DE-A 43 43 891 beschreibt ein Verfahren zur Herstellung von IPDA, wobei IPN in Gegenwart von Ammoniak und einem Suspensions- oder Festbett-Hydrierkatalysator aus der Reihe der Cobalt-, Nickel- und Edelmetallkatalysatoren mit Wasserstoff bei einem Druck von 3 bis 20 MPa und einer Temperatur bis zu 150°C umgesetzt und das erhaltene Reaktionsgemisch destillativ aufgearbeitet wird. Die Reaktion wird zweistufig durchgeführt, wobei genau definierte Temperaturbereiche für die einzelnen Stufen eingehalten werden müssen. Ein cis/trans-Isomerenverhältnis von 80/20 lässt sich in einer Gesamtausbeute an IPDA von 91,9% erzielen.

In dem Verfahren der EP-A 0 926 130 wird die Hydrierung in Gegenwart einer Säure an Katalysatoren durchgeführt, die Kupfer und/oder ein Metall der achten Nebengruppe des Periodensystems enthalten. Es werden sowohl Lewis- als auch Brönstedt-Säuren eingesetzt; bevorzugt wird 2-Ethylhexansäure verwendet. Die Säure-Zugabe bewirkt eine Erhöhung des cis/trans-Isomerenverhältnisses. Die cis/trans-Isomerenverhältnisse sind im allgemeinen ≥ 70/30 bei einer Gesamtausbeute an IPDA ≥ 90%.

Das Verfahren der EP-B 0 729 937 ist dadurch charakterisiert, dass das Verfahren in drei räumlich voneinander getrennten Reaktionsräumen durchgeführt wird, wobei cobalt-, nickel-, ruthenium- und/oder andere edelmetallhaltige Katalysatoren eingesetzt werden. Vor dem zweiten Reaktor wird wäßrige NaOH-Lösung zudosiert, wodurch die Bildung von ringförmigen Nebenprodukten wie 1,3,3-Trimethyl-6-azabicyclo[3,2,1]octan verringert wird.

In dem Verfahren der prioritätsälteren, nicht vorveröffentlichten DE-A 101 42 635.6 wird IPDA mit einem cis/trans-Isomerenverhältnis von mindestens 70/30 ausgehend von IPN gewonnen, indem in dem Hydrierungsschritt ein Hydrierkatalysator mit einem Alkalimetallgehalt ≤ 0,03 Gew.-% - berechnet als Alkalimetalloxid - eingesetzt wird.

Nachteilig an den bekannten Verfahren zur Herstellung von IPDA mit hohem cis-Anteil ist die aufwendige Herstellung der verwendeten Katalysatoren. Zudem unterliegen diese Katalysatoren im allgemeinen einer Alterung, wodurch ihre katalytische Aktivität im Laufe der Zeit abnimmt. Um dies auszugleichen, wird meist die Reaktionstemperatur erhöht, was jedoch zu einer Verschlechterung des cis/trans-Isomerenverhältnisses und der Selektivität und damit zu einer Zunahme der Bildung von Nebenprodukten führt. Die meisten aus dem Stand der Technik bekannten Verfahren zeichnen sich zudem durch eine aufwendige Reaktionsführung aus.

Ein Verfahren zur Herstellung von Isophorondiamin mit einem hohen trans/cis-Isomerenverhältnis ist der DE-A 42 11 454 zu entnehmen. Hierbei wird trans-Isophorondiamin aus Isophoronnitril über das Isophoronnitrilazin hergestellt. Es wird auch beschrieben, dass sich das trans-Isophorondiamin durch Destillation von kommerziell erhältlichem cis/trans-Isomerengemisch gewinnen lassen würde. Da das cis-Isomer jedoch als Hauptprodukt anfällt, ist dieses Verfahren nicht wirtschaftlich.

Aufgabe der Erfindung ist es, ein Verfahren zur Herstellung von Isophorondiamin (IPDA) mit einem cis/trans-Isomerenverhältnis von mindestens 73/27 bereitzustellen, durch das die Nachteile des Standes der Technik vermieden werden.

Die Aufgabe wird erfindungsgemäß gelöst durch ein Verfahren zur Herstellung von im wesentlichen reinem 3-Aminomethyl-3,5,5-trimethylcyclohexylamin (Isophorondiamin, IPDA) mit einem cis/trans-Isomerenverhältnis von mindestens 73/27, welches folgende Schritte enthält:
a) Bereitstellen von Roh-IPDA mit einem cis/trans-Isomerenverhältnis < 73/27;
b) Reinigen und Trennen des Roh-IPDA in eine Fraktion mit einem cis/trans-Isomerenverhältnis von mindestens 73/27 und eine Fraktion mit einem cis/trans-Isomerenverhältnis kleiner als 63/37;
c) Isomerisieren der in Schritt b) erhaltenen Fraktion des im wesentlichen reinen IPDA mit einem cis/trans-Isomerenverhältnis kleiner als 63/37 zu IPDA mit einem cis/trans-Isomerenverhältnis im Bereich von 63/37 bis 66/34 in Gegenwart von H₂, NH₃ und einem Hydrierkatalysator und Rückführen in Schritt a) des Verfahrens.

Durch das erfindungsgemäße Verfahren kann - ausgehend von IPDA mit jedem beliebigen cis/trans-Isomerenverhältnis < 73/27 - IPDA mit einem cis/trans-Isomerenverhältnis ≥ 73/27 gewonnen werden. Das Verfahren bleibt also trotzdem wirtschaftlich, auch wenn durch eine Alterung des Katalysators und einer damit verbundenen Erhöhung der Reaktionstemperatur der cis-Anteil im Roh-IPDA im Laufe der Zeit sinkt. Da das erfindungsgemäße Verfahren unabhängig von solchen Einflüssen ist, ist es den Verfahren überlegen, die IPDA mit einem hohen cis/trans-Isomerenverhältnis durch Einsatz von speziellen, meist in der Herstellung teuren und aufwendigen Katalysatoren herstellen. Unabhängig davon, kann das erfindungsgemäße Verfahren jedoch auch mit derartigen aufwendigeren Syntheseverfahren kombiniert werden. Wirtschaftlicher ist es, das IPDA unter Einsatz von preiswerten Katalysatoren herzustellen und ein schlechteres cis/trans-Isomerenverhältnis des Roh-IPDA in Kauf zu nehmen.

Unter "im wesentlichen reinem" IPDA versteht man IPDA, bei dem der Anteil an Verunreinigungen weniger als 2 Gew.-%, bevorzugt weniger als 1 Gew.-%, besonders bevorzugt weniger als 0,3 Gew.-%, beträgt.

Bevorzugt wird durch das erfindungsgemäße Verfahren IPDA mit einem cis/trans-Isomerenverhältnis im Bereich von 73/27 bis 76/24, besonders bevorzugt mit einem cis/trans-Isomerenverhältnis im Bereich von 73/27 bis 75/25, erhalten. (Siehe auch Diskussion Schritt b).)

Die einzelnen Schritte des Verfahrens werden nun näher erläutert.

### Schritt a)

Im allgemeinen kann jedes Produktgemisch, sogenanntes Roh-IPDA, welches bei einem Verfahren zur Herstellung von IPDA anfällt, eingesetzt werden. "Roh-IPDA" bedeutet, dass das Produktgemisch mindestens 88 Gew.-% IPDA, bevorzugt mindestens 92 Gew.-% IPDA, besonders bevorzugt mindestens 95 Gew.-% IPDA, enthält.

Da IPDA mit einem cis/trans-Isomerenverhältnis von mindestens 73/27 gewonnen werden soll, ist es wirtschaftlich nur sinnvoll, Produktgemische, die IPDA mit einem cis/trans-Isomerenverhältnis kleiner als 73/27 enthalten, einzusetzen. Da die Herstellung von IPDA mit einem cis/trans-Isomerenverhältnis kleiner als 70/30 unter Verwendung von alternden Katalysatoren und ohne aufwendige Verfahrensdurchführung erfolgen kann, ist das erfindungsgemäße Verfahren besonders wirtschaftlich, wenn IPDA mit einem cis/trans-Isomerenverhältnis kleiner als 70/30 eingesetzt wird. Es ist auch möglich, Produktgemische einzusetzen, die IPDA mit einem cis/trans-Isomerenverhältnis größer als 73/27 enthalten, um das cis-Isomer durch Destillation noch weiter anzureichern.

### Schritt b)

Das in Schritt a) bereitgestellte Produktgemisch lässt sich sowohl durch Destillation als auch durch Kristallisation reinigen und trennen.

Cis-IPDA (mit einer Reinheit von 98,9%) hat unter Normaldruck einen Siedepunkt von 253,4°C und einen Schmelzpunkt von 22°C, während trans-IPDA (mit einer Reinheit von 98,4%) unter Normaldruck einen Siedepunkt von 250,7°C und einen Schmelzpunkt von-34,6°C hat. Obwohl sich die Schmelzpunkte der beiden Isomere stärker voneinander unterscheiden als ihre Siedepunkte, ist aus Kostengründen die Reinigung und Trennung durch Destillation bevorzugt.

Diese Reinigung und Trennung des Roh-IPDA durch Destillation kann in jeder beliebigen Destillationskolonne durchgeführt werden. Bevorzugt erfolgt die Destillation in mindestens 2 räumlich voneinander getrennten Kolonnen. Besonders bevorzugt wird wenigstens eine Trennwandkolonne verwendet.

Bei der Destillation des Produktgemisches (Roh-IPDA) werden meist NH₃, sowie leicht- und schwersiedende Komponenten wie beispielsweise die bei der Herstellung von IPDA aus IPN anfallenden Nebenprodukte wie HCN-Eliminierungsprodukte, methylierte Nebenprodukte und/oder unvollständig hydrierte Zwischenprodukte über Kopf bzw. Sumpf der Kolonne entfernt.

Unter leicht-/niedrigsiedenden Komponenten/Verunreinigungen versteht man im Rahmen der Erfindung Komponenten/Verunreinigungen, die niedriger sieden als cis- und trans-IPDA, schwer-/hochsiedende Komponenten/Verunreinigungen sind solche, die höher sieden als cis- und trans-IPDA.

Ferner erfolgt eine Auftrennung in eine an cis-Isomer angereicherte Fraktion und eine an cis-Isomer abgereicherte (und damit an trans-Isomer angereicherte) Fraktion.

Die an cis-angereicherte Fraktion des IPDA weist ein cis/trans-Isomerenverhältnis von mindestens 73/27, bevorzugt ein cis/trans-Isomerenverhältnis im Bereich von 73/27 bis 76/24, besonders bevorzugt ein cis/trans-Isomerenverhältnis im Bereich von 73/27 bis 75/25, auf.

Die an cis-abgereicherte Fraktion des IPDA weist ein cis/trans-Isomerenverhältnis kleiner als 63/37, bevorzugt ≤ 60/40, besonders bevorzugt ≤ 58/42, auf. Die an cis-Isomerangereicherte Fraktion ist kommerziell erwünscht. Die an cis-Isomer abgereicherte Fraktion lässt sich ebenfalls kommerziell verwerten (siehe DE-A 42 11 454).

Wird für die Destillation eine Kolonne eingesetzt, so wird diese im allgemeinen bei Sumpftemperaturen von 150 bis 300°C, bevorzugt von 170 bis 250°C, besonders bevorzugt von 170 bis 185°C, und Kopftemperaturen von 5 bis 100°C, bevorzugt von 10 bis 90°C, besonders bevorzugt von 15 bis 65°C; betrieben. Der Druck in der Kolonne liegt im allgemeinen bei 10 bis 2000 mbar, bevorzugt bei 20 bis 200 mbar, besonders bevorzugt bei 35 bis 50 mbar.

Werden für die Destillation zwei Kolonnen eingesetzt, so wird die erste Kolonne im allgemeinen bei Sumpftemperaturen von 150 bis 300°C, bevorzugt von 170 bis 250°C, besonders bevorzugt von 170 bis 195°C, und Kopftemperaturen von 5 bis 100°C, bevorzugt von 10 bis 90°C, besonders bevorzugt von 15 bis 65°C, betrieben. Der Druck in der ersten Kolonne liegt üblicherweise bei 10 bis 1000 mbar, bevorzugt bei 30 bis 500 mbar, besonders bevorzugt bei 35 bis 200 mbar. Die zweite Kolonne wird im allgemeinen bei Sumpftemperaturen von 140 bis 300°C, bevorzugt von 150 bis 250°C, besonders bevorzugt von 160 bis 195°C, und Kopftemperaturen von 100 bis 250°C, bevorzugt von 130 bis 200°C, besonders bevorzugt von 140 bis 170°C, betrieben. Der Druck in der zweiten Kolonne liegt üblicherweise bei 10 bis 1000 mbar, bevorzugt bei 30 bis 300 mbar, besonders bevorzugt bei 35 bis 120 mbar.

Die Kolonne(n) weist/weisen im allgemeinen eine Trennleistung von insgesamt mindestens 20 theoretischen Böden, bevorzugt von mindestens 30 theoretischen Böden, besonders bevorzugt von mindestens 40 theoretischen Böden, auf.

Die Kolonnen können jeweils verschiedene Einbauten aufweisen. Beispiele für derartige Einbauten sind Füllkörper wie Pall-Ringe und Raschig-Ringe, strukturierte Packungen aus Blech wie Mellapak 250Y® von Sulzer Ltd. (Winterthur/Schweiz), von Montz (Hilden/Deutschland) und von Koch-Glitsch (Wichita, KS/USA) und strukturierte Packungen aus Metallgewebe wie Sulzer BX® von Sulzer Ltd. (Winterthur/Schweiz), von Montz (Hilden/Deutschland) und von Koch-Glitsch (Wichita, KS/USA).

### Schritt c)

Erfindungsgemäß wird die an cis-Isomer abgereicherte Fraktion - nach Isomerisierung - in Schritt a) des Verfahrens rückgeführt. Dieser Isomerisierungsschritt, - nämlich, dass sich bei Leiten von IPDA mit einem beliebigen cis/trans-Isomerenverhältnis in Gegenwart von H₂ und NH₃ über einen Hydrierungskatalysator temperaturunabhängig ein thermodynamisches Gleichgewicht mit einem cis/trans-Isomerenverhältnis im Bereich von 63/37 bis 66/34, bevorzugt von 64/36 bis 66/34, besonders bevorzugt von 64/36 bis 65/35, einstellt, - ist aus dem Stand der Technik nicht bekannt.

Die Isomerisierung von IPDA mit einem cis/trans-Isomerenverhältnis größer als 63/37 ist zwar möglich, wäre aber wirtschaftlich unsinnig, wenn primäres Ziel die Erhöhung des cis-Anteils im cis/trans-Isomerengemisch ist.

Die Isomerisierung von IPDA mit einem cis/trans-Isomerenverhältnis kleiner als 63/37 zu IPDA mit einem cis/trans-Isomerenverhältnis im Bereich von 63/37 bis 66/34 wird im allgemeinen bei Temperaturen von 70 bis 200°C, bevorzugt von 80 bis 150°C, besonders bevorzugt von 90 bis 130°C, und Drücken von 10 bis 300 bar, bevorzugt von 50 bis 250 bar, besonders bevorzugt von 100 bis 240 bar, durchgeführt. Die Reaktionsdauer ist abhängig von der Isomerisierungstemperatur und dem eingesetzten Katalysator.

In dem erfindungsgemäßen Schritt c) zur Isomerisierung von IPDA können als Hydrierkatalysatoren prinzipiell alle gängigen Hydrierkatalysatoren eingesetzt werden, bevorzugt solche, die mindestens ein Übergangsmetall ausgewählt aus der Gruppe Kupfer, Silber, Gold, Eisen, Cobalt, Nickel, Rhenium, Ruthenium, Rhodium, Palladium, Osmium, Iridium, Platin, Chrom, Molybdän und Wolfram, jeweils in metallischer Form (Oxidationsstufe 0) oder in Form von Verbindungen wie z. B. Oxiden enthalten, die unter den Verfahrensbedingungen zum entsprechenden Metall reduziert werden.

Von diesen Hydrierkatalysatoren sind besonders solche bevorzugt, die mindestens ein Übergangsmetall ausgewählt aus der Gruppe Kupfer, Silber, Eisen, Cobalt, Nickel, Ruthenium, Rhodium, Palladium, Osmium, Iridium und Platin, jeweils in metallischer Form (Oxidationsstufe 0) oder in Form von Verbindungen wie z. B. Oxiden enthalten, die unter den Verfahrensbedingungen zum entsprechenden Metall reduziert werden.

Ganz besonders bevorzugt sind Hydrierkatalysatoren, die mindestens ein Übergangsmetall ausgewählt aus der Gruppe Kupfer, Cobalt, Nickel, Ruthenium, Iridium, Rhodium, Palladium und Platin, jeweils in metallischer Form (Oxidationsstufe 0) oder in Form von Verbindungen wie z. B. Oxiden enthalten, die unter den Verfahrensbedingungen zum entsprechenden Metall reduziert werden.

Am meisten bevorzugt sind Hydrierkatalysatoren, die mindestens ein Übergangsmetall ausgewählt aus der Gruppe Cobalt und Ruthenium, jeweils in metallischer Form (Oxidationsstufe 0) oder in Form von Verbindungen wie z. B. Oxiden enthalten, die unter den Verfahrensbedingungen zum entsprechenden Metall reduziert werden.

Sind die katalytisch aktiven Übergangsmetalle auf Trägem - ausgewählt aus der Gruppe Aluminiumoxid (Al₂O₃), Zirkoniumdioxid (ZrO₂), Titandioxid (TiO₂), Kohlenstoff und/oder sauerstoffhaltige Verbindungen des Siliciums, berechnet als SiO₂, - aufgebracht, so enthalten diese Katalysatoren im allgemeinen insgesamt - bezogen auf das Gesamtgewicht des Katalysators - von 20 bis 99,9 Gew.-%, bevorzugt von 30 bis 99,9 Gew.-%, besonders bevorzugt von 40 bis 99,9 Gew.-%, Träger und von 0,1 bis 80 Gew.-%, bevorzugt von 0,1 bis 70 Gew.-%, besonders bevorzugt von 0,1 bis 60 Gew.-%, Übergangsmetall, berechnet als Metall in der Oxidationsstufe 0.

Bei der Isomerisierung hält man eine Katalysatorbelastung von 0,1 bis 2, vorzugsweise von 0,2 bis 1,5, besonders bevorzugt von 0,4 bis 1, kg IPDA mit einem cis/trans-Isomerenverhältnis kleiner als 63/37 pro 1 Katalysator und Stunde ein.

Die Isomerisierung wird vorzugsweise in flüssigem Ammoniak durchgeführt. Pro mol IPDA mit einem cis/trans-Isomerenverhältnis kleiner als 63/37 setzt man bei der Isomerisierung zweckmäßig einen Überschuß von 0,5 bis 100 mol, bevorzugt von 2 bis 50 mol, besonders bevorzugt von 5 bis 40 mol, NH₃ ein.

Die Isomerisierung des IPDA kann in Anwesenheit eines Lösungsmittels, wie z. B. Alkanolen oder Ethern (Tetrahydrofuran) durchgeführt werden, man kann aber auch ohne Zusatz eines Lösungsmittels arbeiten.

Die Isomerisierung lässt sich sowohl kontinuierlich als auch diskontinuierlich durchführen. Die kontinuierliche Reaktionsführung ist bevorzugt. Es können beliebige druckfeste Rührbehälter oder Rührbehälterkaskaden eingesetzt werden. In einer besonders bevorzugten Ausführungsform werden Reaktoren eingesetzt, in denen das IPDA mit einem cis/trans-Isomerenverhältnis kleiner als 63/37 kontinuierlich in Sumpf- oder Rieselfahrweise über ein fest angeordnetes Katalysatorbett geleitet wird. Man kann auch Schachtöfen einsetzen.

In einer anderen Ausführungsvariante des erfindungsgemäßen Isomerisierungsverfahrens wird die Isomerisierung nicht in einem separaten Rührkessel unter den oben angegebenen Bedingungen durchgeführt, sondern das zu isomerisierende IPDA mit einem cis/trans-Isomerenverhältnis < 63/37 wird einer beliebigen, aus dem Stand der Technik bekannten Reaktion zur Herstellung von IPDA aus IPN, H₂ und NH₃ in Gegenwart eines Hydrierkatalysators zugeführt. Beispiele für Herstellungsverfahren von IPDA aus dem Stand der Technik sind in der Einleitung gegeben.

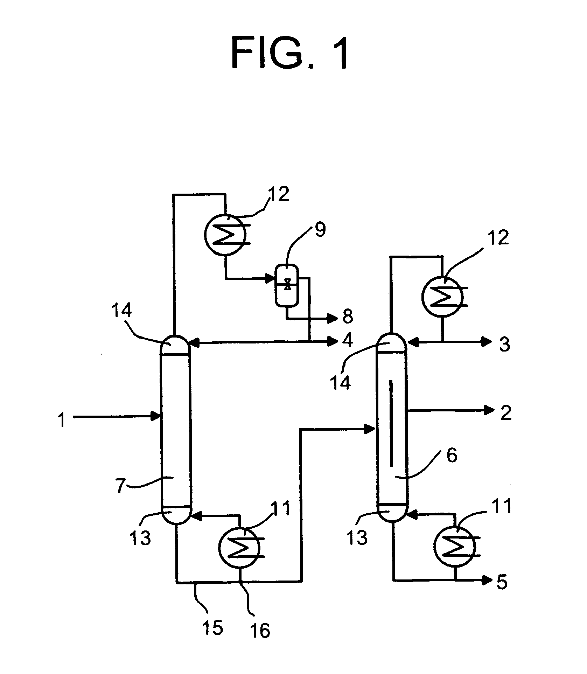

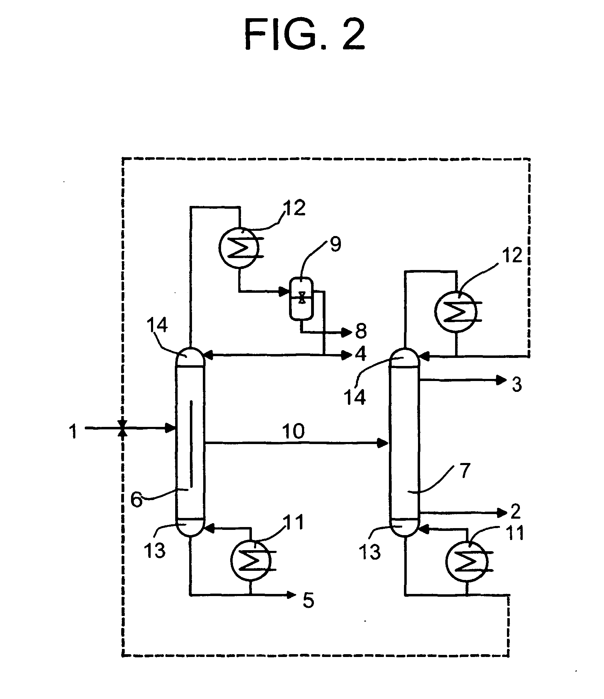

Die anliegende Zeichnung zeigt in den Figuren 1 und 2 schematisch Anlagen, in denen Schritt b) des erfindungsgemäßen Verfahrens zur Gewinnung von IPDA mit einem cis/trans-Isomerenverhältnis von mindestens 73/27 in zwei Kolonnen durchgeführt wird, nämlich in
- **Figur 1**: eine schematische Darstellung einer Anlage, in der die erste Kolonne eine herkömmliche Destillationskolonne und die zweite Kolonne eine Trennwandkolonne ist.
- **Figur 2**: eine schematische Darstellung einer Anlage, wobei die erste Kolonne eine Trennwandkolonne und die zweite Kolonne eine herkömmliche Destillationskolonne ist.

In einer Anlage gemäss Figur 1 wird ein Produktgemisch enthaltend Roh-IPDA über einen Einlass 1 in eine herkömmliche Destillationskolonne 7 gegeben und dort destilliert. Leichtsiedende Komponenten werden über Kopf 14 der Kolonne abgezogen, nach Kondensation in einem Kondensator 12 in einen Phasenabscheider 9 überführt und dort in eine leichtere organische und eine schwerere wäßrige Phase getrennt. Die leichtere organische Phase wird teilweise über den Abzug 4 verworfen, teilweise in die Destillationskolonne 7 rückgeführt. Die schwerere wäßrige Phase wird über die Ableitung 8 entsorgt.

Die Temperaturen am Kopf der Destillationskolonne 7 betragen im allgemeinen 20 bis 100°C, bevorzugt 30 bis 80°C und besonders bevorzugt 35 bis 65°C bei einem durchschnittlichen Druck in der Kolonne von 50 bis 1000 mbar. Die Temperaturen am Sumpf der Destillationskolonne 7 betragen im allgemeinen 150 bis 250°C, bevorzugt 170 bis 225°C, besonders bevorzugt 170 bis 190°C. Bevorzugt ist ein durchschnittlicher Druck in der Kolonne von 100 bis 500 mbar, besonders bevorzugt ist ein durchschnittlicher Druck von 110 bis 200 mbar.

Der Sumpf 13 der Destillationskolonne 7 wird kontinuierlich in eine Trennwandkolonne 6 überführt. In Leitung 15 führt eine Verzweigung 16 zu einem Verdampfer 11, wo ein Teil des Sumpfablaufs wieder verdampft und in Kolonne 7 rückgeführt wird. Die an cis-Isomer angereicherte Fraktion wird über einen Seitenabzug 2 der Kolonne 6 entnommen, die an trans-Isomer angereicherte Fraktion wird über Kopf der Destillationskolonne abgezogen, in einem Kondensator 12 kondensiert und anschließend teilweise in Kolonne 6 rückgeführt, teilweise über Leitung 3 entnommen. Nach Isomerisierung - und damit verbundene Erhöhung des cis-Anteils - wird die so isomerisierte Fraktion in Leitung 1 eingespeist. Über den Sumpf 13 der Trennwandkolonne 6 werden hochsiedende Verunreinigungen teils über Leitung 5 ausgeschleust, teils nach Verdampfung in einem Verdampfer 11 der Kolonne 6 wieder zugeführt.

Die Trennwandkolonne 6 wird im allgemeinen bei Temperaturen von 100 bis 250°C am Kopf und Temperaturen von 150 bis 300°C am Sumpf und Drücken von 10 bis 1000 mbar, bevorzugt bei Temperaturen von 130 bis 190°C am Kopf und/oder Temperaturen von 170 bis 250°C am Sumpf und/oder Drücken von 30 bis 200 mbar, besonders bevorzugt bei Temperaturen von 140 bis 160°C am Kopf und/oder Temperaturen von 170 bis 195°C am Sumpf und/oder Drücken von 35 bis 50 mbar, betrieben.

Wird Schritt b) des erfindungsgemäße Verfahrens in einer Anlage gemäss Figur 2 durchgeführt, so wird das Roh-IPDA über die Zufuhr 1 in eine Trennwandkolonne 6 geschleust. Hochsiedende Verunreinigungen werden als Sumpf 13 der Kolonne teils über die Ableitung 5 ausgeschleust, teils nach Verdampfung in einem Verdampfer 11 der Kolonne wieder zugeführt. Leichtsiedende Verunreinigungen werden über Kopf 14 der Kolonne abgezogen und nach Kondensation in einem Kondensator 12 in einen Phasenscheider 9 überführt. Die sich abgesetzte leichtere organische Phase wird teilweise über die Ableitung 4 ausgeschleust, teilweise in die Trennwandkolonne 6 rückgeführt. Die schwerere Phase wird über die Ableitung 8 ausgeschleust.

Das gereinigte IPDA wird über einen Seitenabzug 10 der Trennwandkolonne 6 entnommen und in eine weitere Kolonne 7 überführt, die hier als gewöhnliche Destillationskolonne ausgebildet ist. Die leichtestsiedenden Komponenten werden über Kopf 14 der Kolonne 7 abgezogen und nach Kondensation in einem Kondensator 12 teils der Kolonne 7 wieder zugeführt, teils in die Zuleitung 1 eingeschleust, um sie einer erneuten Trennung in Kolonne 6 zuzuführen. Dasselbe geschieht mit den schwerstsiedenden Komponenten, die über den Sumpf 13 der Kolonne 7 abgezogen und teilweise nach Verdampfung in einem Verdampfer 11 wieder in Kolonne 7 eingespeist, teils dem Produktgemisch in Zuleitung 1 zugegeben werden.

Die an cis-Isomer angereicherte Fraktion wird über einen Seitenabzug 2, die an trans-Isomer angereicherte Fraktion über einen Seitenabzug 3 abgezogen und - nach Isomerisierung - in Leitung 1 gespeist. Der Seitenabzug für die an cis-Isomer angereicherte Fraktion liegt unterhalb des Seitenabzugs für die an trans-Isomer angereicherte Fraktion.

Die Durchführung von Schritt b) des Verfahrens in einer Anlage gemäß Fig. 2 ist besonders vorteilhaft, da an jeweils zwei Stellen leicht- und schwersiedende Verunreinigungen abgetrennt werden: Leichtsiedende Komponenten werden sowohl über Kopf 14 der Kolonne 6, als auch über Kopf 14 der Kolonne 7 abgetrennt, während schwersiedende Verunreinigungen sowohl über den Sumpfablauf 13 der Kolonne 6 als auch über den Sumpfablauf 13 der Kolonne 7 abgetrennt werden.

In einer Anlage gemäß Figur 2 wird die Trennwandkolonne 6 im allgemeinen bei Temperaturen von 5 bis 100°C, bevorzugt von 10 bis 90°C, besonders bevorzugt von 15 bis 50°C betrieben. Die Temperaturen am Sumpf betragen in der Regel 150 bis 300°C, bevorzugt 170 bis 250°C, besonders bevorzugt 170 bis 195°C. Der durchschnittliche Druck in Trennwandkolonne 6 liegt bei 10 bis 1000 mbar, bevorzugt bei 30 bis 200 mbar, besonders bevorzugt bei 35 bis 50 mbar. Die Temperaturen am Kopf der Destillationskolonne 7 liegen im allgemeinen bei 130 bis 250°C, bevorzugt bei 140 bis 200°C, besonders bevorzugt bei 150 bis 170°C. Die Temperaturen am Sumpf der Destillationskolonne 7 betragen in der Regel 140 bis 250°C, bevorzugt 150 bis 220°C, besonders bevorzugt 160 bis 190°C. Der durchschnittliche Druck in der Destillationskolonne 7 liegt in einer Anlage gemäß Figur 2 bei 30 bis 1000 mbar, bevorzugt bei 50 bis 300 mbar, besonders bevorzugt bei 80 bis 120 mbar.

Die Erfindung wird nun in den folgenden Ausführungsbeispielen zusätzlich näher erläutert.

### Ausführungsbeispiele

### Vergleichsbeispiel 1:Herstellung von IPDA aus IPN und anschließender destillativer Aufarbeitung

Die aminierende Hydrierung von Isophoronnitril zu Isophorondiamin erfolgt in einem kontinuierlichen Prozess in drei hintereinander geschalteten Reaktoren bei einem Druck von 250 bar, wie in EP-B 0 729 937 beschrieben. Der Katalysator wird unter Wasserstoffatmosphäre mit einer Heizrate von 2K/min bis zu 280°C hochgeheizt. Nach 12 h Halten bei dieser Temperatur wird auf die jeweilige Reaktionstemperatur zurückgefahren.

Durch den ersten Reaktor (Iminierungsreaktor 200 ml), gefüllt mit γ-Al₂O₃ (4 mm-Stränge) als Träger für den Katalysator, fährt man in Sumpffahrweise Isophoronnitril (130 ml/h), Ammoniak (600 g/h) und Wasserstoff (300 l/h) bei einer Temperatur von 80 bis 100°C. Dort erfolgt die Iminierung. Das Reaktionsgemisch wird in den ersten Reaktor mit dem in der EP-A 0 742 045 beschriebenen Katalysator gefahren. Dort ist die Temperatur 90°C. Im letzten Reaktor (130°C) erfolgt die Nachhydrierung am gleichen Katalysator. Die Reihenfolge ist Sumpf-Riesel-Sumpf. Das Produktgemisch wird in einen Abscheider entspannt (Zusammensetzung siehe Tabelle 1) und diskontinuierlich in einer Destillationskolonne aufdestilliert. Daten der Destillationskolonne: Kolonnen-Durchmesser: 30 mm, Packungshöhe: 1,5 m, Packung: Sulzer DX von Sulzer Ltd. (Winterthur, Schweiz), 45 theoretische Trennstufen, Druck 30 mbar, Rücklaufverhältnis 10/1.

Als erstes wird eine Fraktion abgetrennt mit einem cis/trans-Isomerenverhältnis von 60/40. Der Siedepunkt dieser Fraktion beträgt 137,5°C. Anschließend wird eine Fraktion isoliert, die 75% cis-IPDA und 25% trans-IPDA enthält und einen Siedepunkt von 138,7°C aufweist. Entnommene Proben werden jeweils gaschromatographisch analysiert.

### Beispiel 2: Herstellung von IPDA aus IPN, anschließender destillativer Aufarbeitung und Rückführung der an cis-abgereicherten Fraktion in den Synthesereaktor

Beispiel 1 wurde wiederholt, jedoch wurden von der zuerst abgetrennten Fraktion (IPDA mit einem cis/trans-Isomerenverhältnis von 60/40) je 50 ml/h zusätzlich zum IPN-Strom (130 ml/h) in die Reaktion eingespeist. Entnommene Proben wurden gaschromatographisch analysiert. Die Zusammensetzung des Roh-IPDA ist der Tabelle 1 zu entnehmen.

**Tabelle 1: Ergebnisse der Beispiele 1 und 2**

| | **Vergleichsbeispiel 1** | **Beispiel 2** |
|---|---|---|
| Gesamtausbeute an IPDA | 92,8% | 94,4% |
| Cis/trans-Isomerenverhältnis des Roh-IPDA | 69,7 Gew.-% | 66,1 Gew.-% |
| Nebenprodukte durch HCN-Eliminierung (Ia, Ib) | 4,4 Gew.-% | 2,7 Gew.-% |
| methylierte Nebenprodukte (IIa, IIb) | 0,8 Gew.-% | 0,4 Gew.-% |
| cyclisches Nebenprodukt 1,3,3-Trimethyl-6-azabicyclo[3,2,1]octan (IV) | 0,3 Gew.-% | 0,5 Gew.-% |
| Aminonitril (III) | 0 Gew.-% | 0,1 Gew.-% |
| Gesamtanteil an Nebenprodukten im Roh-IPDA | 5,5 Gew.-% | 3,7 Gew.-% |

| | | |
|---|---|---|
| 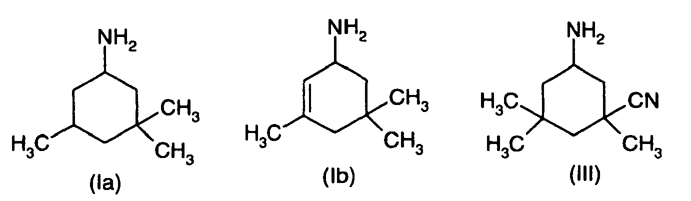 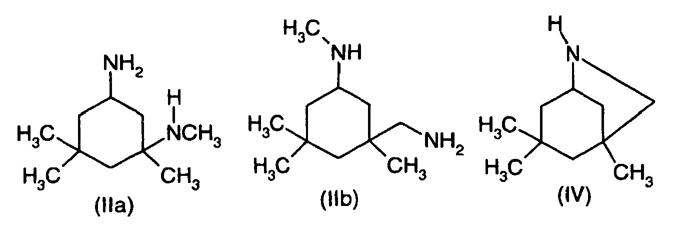 | | |

Durch die erfindungsgemäße Abtrennung der an cis-Isomer abgereicherten IPDA-Fraktion und die Rückführung in den Synthesereaktor zur Isomerisierung kann die Gesamtausbeute an IPDA noch weiter gesteigert, und der Anteil der Nebenprodukte gesenkt werden. Eine leichte Zunahme des Anteils an Verbindung IV ist unschädlich, da diese leicht von IPDA abtrennbar ist. Das cis/trans-Isomerenverhältnis wird zwar geringfügig schlechter, doch ist diese Vorgehensweise insgesamt trotzdem wirtschaftlicher als der Einsatz von teuren Katalysatoren und Beachtung einer aufwendigen Reaktionsführung zur Herstellung eines IPDA mit einem von vornherein höheren cis/trans-Isomerenverhältnis, da das cis-Isomer durch das anschließende destillative Verfahren angereichert werden kann.

### Beispiel 3: Isomerisierung von trans-IPDA

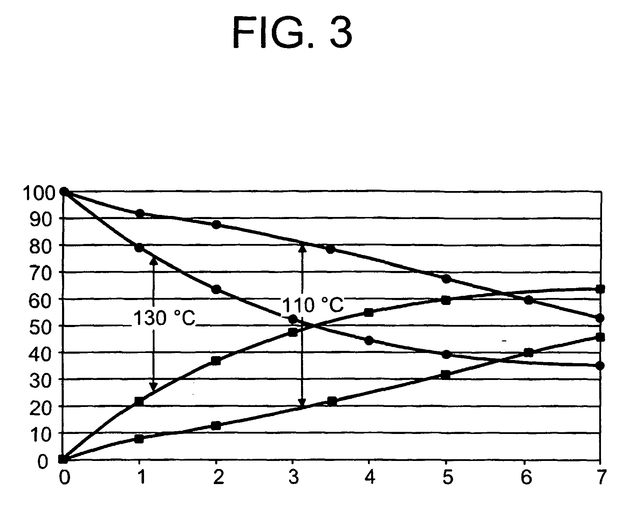

In einem 300 ml Autoklaven mit Magnetrührer und Katalysatorkorb wurden 20 ml eines gemäß der EP-A 0 742 045 hergestellten Cobalt-Katalysators und 40 ml trans-Isophorondiamin vorgelegt. Der Autoklav wurde verschlossen, und es wurden 60 ml Ammoniak über Schauglas aufgepresst. Mit Wasserstoff wurde ein Druck von 50 bar eingestellt. Nach Erwärmen auf eine Reaktionstemperatur von 130°C wurde durch erneutes Aufpressen mit Wasserstoff ein Druck von 250 bar eingestellt. Der Autoklav wurde 24 Stunden bei diesen Bedingungen gehalten. Nach Versuchsende wurde der Autoklav 6 Stunden unter Rühren und bei Raumtemperatur entspannt, um noch gelösten Ammoniak ausgasen zu lassen. Für die gaschromatographische Analytik wurden Druckproben entnommen. Die Ergebnisse sind graphisch in Figur 3 dargestellt.

### Beispiel 4: Isomerisierung von trans-IPDA

Beispiel 3 wurde wiederholt, jedoch wurde eine Reaktionstemperatur von 110°C statt 130°C eingestellt. Die Ergebnisse sind graphisch in Figur 3 dargestellt.

### Wertung der Figur 3

Figur 3 zeigt die graphische Auswertung der Ausführungsbeispiele 3 und 4. Auf der y-Achse ist der Anteil an cis-IPDA in einem cis-/trans-IPDA-Isomerengemisch in Gew.-% angegeben. Auf der x-Achse ist die Reaktionszeit in Stunden aufgetragen. cis-IPDA ist durch ausgefüllte Kästchen dargestellt, trans-IPDA durch ausgefüllte Kreise.

Man sieht, dass ausgehend von 100 Gew.-%-igem trans-IPDA im Laufe der Zeit der Anteil des trans-Isomeren kontinuierlich auf einen Wert von ca. 35 Gew.-% abnimmt, während der Anteil des cis-Isomeren kontinuierlich auf einen Wert von ca. 65 Gew.-% ansteigt. Bei einer Reaktionstemperatur von 130°C ist die Gleichgewichtseinstellung bereits nach 7 Stunden beendet, während sich bei einer Erniedrigung der Reaktionstemperatur auf 110°C die Reaktionszeit auf 23 Stunden verlängert. Bei 110°C wird daher nach 7 Stunden ein Isomerengemisch mit ca. 45 Gew.-% cis und ca. 55 Gew.-% trans erhalten.

### Beispiel 5: Isomerisierung von cis-IPDA

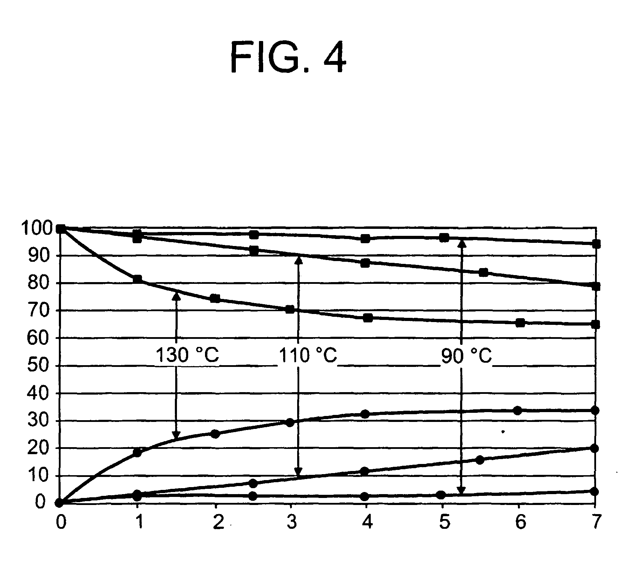

In einem 300 ml Autoklaven mit Magnetrührer und Katalysatorkorb wurden 20 ml gemäß der EP-A 0 742 045 hergestellter Cobalt-Katalysator und 40 ml cis-IPDA vorgelegt. Der Autoklav wurde verschlossen, und es wurden 60 ml Ammoniak über Schauglas aufgepresst. Mit Wasserstoff wurde ein Druck von 50 bar eingestellt. Nach schrittweisem Erwärmen auf eine Reaktionstemperatur von 130°C wurde mit Wasserstoff ein Druck von 250 bar eingestellt. Der Autoklav wurde 24 Stunden bei diesen Bedingungen gehalten. Nach Versuchsende wurde der Autoklav 6 Stunden unter Rühren und bei Raumtemperatur entspannt, um noch gelösten Ammoniak ausgasen zu lassen. Für die gaschromatographische Analytik wurden Druckproben entnommen. Die Ergebnisse sind der Figur 4 zu entnehmen.

### Beispiel 6: Isomerisierung von cis-IPDA

Beispiel 5 wurde wiederholt, jedoch wurde eine Reaktionstemperatur von 110°C statt 130°C eingestellt. Die Ergebnisse sind graphisch in Figur 4 dargestellt.

### Beispiel 7: Isomerisierung von cis-IPDA

Beispiel 5 wurde wiederholt, jedoch wurde eine Reaktionstemperatur von 90°C statt 130°C eingestellt. Die Ergebnisse sind der Figur 4 zu entnehmen.

### Wertung von Figur 4

Figur 4 zeigt die graphische Auswertung der Ausführungsbeispiele 5, 6 und 7. Auf der x-Achse ist die Reaktionszeit in Stunden aufgetragen. Auf der γ-Achse ist der Anteil an cis-IPDA in einem cis-/trans-IPDA-Isomerengemisch in Gew.-% angegeben. cis-IPDA ist durch ausgefüllte Kästchen dargestellt, trans-IPDA durch ausgefüllte Kreise.

Man sieht, dass ausgehend von 100 Gew.-%-igem cis-IPDA im Laufe der Zeit der Anteil des cis-Isomeren kontinuierlich auf einen Wert von ca. 65 Gew.-% abnimmt, während der Anteil des trans-Isomeren kontinuierlich auf einen Wert von ca. 35 Gew.-% ansteigt. Die Gleichgewichtseinstellung erfolgt je nach Reaktionstemperatur verschieden schnell. Bei einer Reaktionstemperatur von 130°C ist die Gleichgewichtseinstellung bereits nach 7 Stunden beendet, während sich bei einer Erniedrigung der Reaktionstemperatur auf 110°C die Reaktionszeit auf 22 Stunden verlängert und bei weiterer Erniedrigung der Reaktionstemperatur auf 90°C 3 Tage benötigt werden.

### Bezugszeichenliste

- 1: Zufuhr des Roh-IPDA
- 2: Abzug für die an cis-Isomer angereicherte Fraktion
- 3: Abzug für die an trans-Isomer angereicherte Fraktion
- 4: Abzug für leichtsiedende Verunreinigungen
- 5: Abzug für hochsiedende Verunreinigungen
- 6: Trennwandkolonne
- 7: herkömmliche Destillationskolonne
- 8: Abzug für die schwereren Bestandteile der Leichtsieder-Fraktion
- 9: Phasenabscheider
- 10: Seitenabzug
- 11: Verdampfer
- 12: Kondensator
- 13: Sumpf der Kolonne
- 14: Kopf der Kolonne
- 15: Leitung
- 16: Verzweigung

## Patentansprüche

1. Verfahren zur Herstellung von im wesentlichen reinem 3-Aminomethyl-3,5,5-Trimethylcyclohexylamin (Isphorondiamin, IPDA) mit einem cis/trans-Isomerenverhältnis von mindestens 73/27 enthaltend folgende Schritte:
a) Bereitstellen von Roh-IPDA mit einem cis/trans-Isomerenverhältnis < 73/27;
b) Reinigen und Trennen des Roh-IPDA in eine Fraktion mit einem cis/trans-Isomerenverhältnis von mindestens 73/27 und eine Fraktion mit einem cis/trans-Isomerenverhältnis kleiner als 63/37;
c) Isomerisieren der in Schritt b) erhaltenen Fraktion des im wesentlichen reinen IPDA mit einem cis/trans-Isomerenverhältnis kleiner als 63/37 zu IPDA mit einem cis/trans-Isomerenverhältnis im Bereich von 63/37 bis 66/34 in Gegenwart von H₂, NH₃ und einem Hydrierkatalysator und Rückführen in Schritt a) des Verfahrens.

2. Verfahren nach Anspruch 1, **dadurch gekennzeichnet, dass** das Reinigen und Trennen des Roh-IPDA in Schritt b) des Verfahrens durch Destillation erfolgt.

3. Verfahren nach Anspruch 2, **dadurch gekennzeichnet, dass** Schritt b) des Verfahrens in mindestens zwei räumlich voneinander getrennten Destillationskolonnen durchgeführt wird.

4. Verfahren nach Anspruch 3, **dadurch gekennzeichnet, dass** von den Destillationskolonnen mindestens eine eine Trennwandkolonne ist.

5. Verfahren nach einem der Ansprüche 1 bis 4, **dadurch gekennzeichnet, dass** in Schritt b) des Verfahrens das IPDA in eine Fraktion mit einem cis/trans-Isomerenverhältnis im Bereich von 73/27 bis 76/24 und eine Fraktion mit einem cis/trans-Isomerenverhältnis kleiner als 63/37 getrennt wird.

6. Verfahren nach einem der Ansprüche 1 bis 5, **dadurch gekennzeichnet, dass** in Schritt a) des Verfahrens Roh-IPDA mit einem cis/trans-Isomerenverhältnis ≤ 70/30 bereitgestellt wird.

7. Verfahren nach einem der Ansprüche 1 bis 6, **dadurch gekennzeichnet, dass** in Schritt c) des Verfahrens als Hydrierkatalysator ein Katalysator enthaltend mindestens ein Übergangsmetall ausgewählt aus der Gruppe Kupfer, Silber, Gold, Eisen, Cobalt, Nickel, Rhenium, Ruthenium, Rhodium, Palladium, Osmium, Iridium, Platin, Chrom, Molybdän und Wolfram, bevorzugt ausgewählt aus der Gruppe Kupfer, Silber, Eisen, Cobalt, Nickel, Ruthenium, Rhodium, Palladium, Osmium, Iridium und Platin, besonders bevorzugt ausgewählt aus der Gruppe Kupfer, Cobalt, Nickel, Ruthenium, Iridium, Rhodium, Palladium und Platin, eingesetzt wird.

## Claims

1. A process for preparing substantially pure 3-aminomethyl-3,5,5-trimethylcyclohexylamine (isophoronediamine, IPDA) having a cis/trans isomer ratio of at least 73/27, comprising the following steps:
a) providing crude IPDA having a cis/trans isomer ratio of < 73/27;
b) purifying and separating the crude IPDA into a fraction having a cis/trans isomer ratio of at least 73/27 and a fraction having a cis/trans isomer ratio of less than 63/37;
c) isomerizing the fraction of substantially pure IPDA having a cis/trans isomer ratio of less than 63/37 obtained in step b) to IPDA having a cis/trans isomer ratio in the range from 63/37 to 66/34 in the presence of H₂, NH₃ and a hydrogenation catalyst and recycling it into step a) of the process.

2. The process according to claim 1, wherein the crude IPDA is purified and separated in step b) of the process by distillation.

3. The process according to claim 2, wherein step b) of the process is carried out in two spatially separated distillation columns.

4. The process according to claim 3, wherein at least one of the distillation columns is a dividing wall column.

5. The process according to any of claims 1 to 4, wherein the IPDA is separated in step b) of the process into a fraction having a cis/trans isomer ratio in the range from 73/27 to 76/24 and a fraction having a cis/trans isomer ratio of less than 63/37.

6. The process according to any of claims 1 to 5, wherein crude IPDA having a cis/trans isomer ratio of ≤ 70/30 is provided in step a) of the process.

7. The process according to any of claims 1 to 6, wherein the hydrogenation catalyst used in step c) of the process is a catalyst comprising at least one transition metal selected from the group of copper, silver, gold, iron, cobalt, nickel, rhenium, ruthenium, rhodium, palladium, osmium, iridium, platinum, chromium, molybdenum and tungsten, preferably selected from the group of copper, silver, iron, cobalt, nickel, ruthenium, rhodium, palladium, osmium, iridium and platinum, more preferably selected from the group of copper, cobalt, nickel, ruthenium, iridium, rhodium, palladium and platinum.

## Revendications

1. Procédé de préparation de 3-aminométhyl-3,5,5-triméthylcyclohexylamine (isophoronediamine, IPDA) essentiellement pur présentant un rapport isomérique cis/trans d'au moins 73/27, comprenant les étapes suivantes consistant à :
a)préparer de l'IPDA brute présentant un rapport isomérique cis/trans < 73/27 ;
b)purifier et séparer l'IDPA brute en une fraction présentant un rapport isomérique cis/trans d'au moins 73/27 et une fraction présentant un rapport isomérique cis/trans inférieur à 63/37 ;
c)isomériser la fraction, obtenue dans l'étape b), de l'IPDA essentiellement pure présentant un rapport isomérique cis/trans inférieur à 63/37 pour donner de l'IPDA présentant un rapport isomérique cis/trans dans la plage de 63/37 à 66/34 en présence de H₂, NH₃ et d'un catalyseur d'hydrogénation et remise en circulation dans l'étape a) du procédé.

2. Procédé selon la revendication 1, **caractérisé en ce que** la purification et la séparation de l'IPDA brute dans l'étape b) du procédé a lieu par distillation.

3. Procédé selon la revendication 2, **caractérisé en ce que** l'étape b) du procédé est mise en oeuvre dans au moins deux colonnes à distiller séparées spatialement l'une de l'autre.

4. Procédé selon la revendication 3, **caractérisé en ce qu'**au moins une des colonnes à distiller est une colonne à paroi de séparation.

5. Procédé selon l'une quelconque des revendications 1 à 4, **caractérisé en ce que** l'IPDA est séparée dans l'étape b) du procédé en une fraction présentant un rapport isomérique cis/trans dans la plage de 73/27 à 76/24 et une fraction présentant un rapport isomérique cis/trans inférieur à 63/37.

6. Procédé selon l'une quelconque des revendications 1 à 5, **caractérisé en ce que** de l'IPDA brute présentant un rapport isomérique cis/trans ≤ 70/30 est préparée dans l'étape a) du procédé.

7. Procédé selon l'une quelconque des revendications 1 à 6, **caractérisé en ce qu'**un catalyseur contenant au moins un métal de transition choisi dans le groupe cuivre, argent, or, fer, cobalt, nickel, rhénium, ruthénium, rhodium, palladium, osmium, iridium, platine, chrome, molybdène et tungstène, choisi de manière préférée dans le groupe cuivre, argent, fer, cobalt, nickel, ruthénium, rhodium, palladium, osmium, iridium et platine, choisi de manière particulièrement préférée dans le groupe cuivre, cobalt, nickel, ruthénium, iridium, rhodium, palladium et platine, est utilisé comme catalyseur d'hydrogénation dans l'étape c) du procédé.
